# EUROPÄISCHE PATENTSCHRIFT

(11) **EP 1 220 672 B1**
(45) Veröffentlichungstag und Bekanntmachung des Hinweises auf die Patenterteilung: **04.10.2006**
(21) Anmeldenummer: 00943518.1
(22) Anmeldetag: 27.07.2000
(51) Int. Cl.: A61K 31/505, A61K 31/519, A61P 25/00

(54) **ARZNEIMITTEL ZUR BEHANDLUNG VON NEUROPATHIEN**
MEDICAMENT FOR TREATMENT OF NEUROPATHIES
PRODUITS PHARMACEUTIQUES UTILISES POUR LE TRAITEMENT DE NEUROPATHIES

(30) Priorität: 12.10.1999 CH 186299
(43) Veröffentlichungstag der Anmeldung: 10.07.2002
(73) Patentinhaber: Lilly Icos LLC, Wilmington, DE 19801 (US)
(72) Erfinder: Lilly Icos LLC, Wilmington, DE 19801 (US)
(74) Vertreter: Ritscher, Thomas
(86) Internationale Anmeldenummer: PCT/CH2000/000409
(87) Internationale Veröffentlichungsnummer: WO 2001/026659

(56) Entgegenhaltungen:
- WO-A-93/07149
- US-A- 4 666 908
- US-A- 6 075 028
- M. S. RENDELL ET AL : "Sildenafil for treatment of erectile dyfunction in men with diabetes" JAMA, THE JOURNAL OF THE AMERICAN MEDICAL ASSOCIATION, Bd. 281, Nr. 5, Februar 1999 (1999-02), Seiten 421-426, XP000986570
- H. G. NURNBERG ET AL : "Sildenafil for iatrogenic serotonergic antidepressant medication-induced sexual dysfunction in 4 patients" JOURNAL OF CLINICAL PSYCHIATRY., Bd. 60, Nr. 1, Januar 1999 (1999-01), Seiten 33-35, XP000987161 XX, XX ISSN: 0160-6689
- M. BREWER ET AL : "Sildenafil citrate therapy in men with Parkinson's diease" MOVEMENT DISORDERS, Bd. 13, Nr. 5, September 1998 (1998-09), Seite 860 XP000991091
- T. A. ZESIEWICZ ET AL : "Sildenafil citrate (Viagra) for the treatment of erectile dysfunction in men with Parkinson's disease" NEOROLOGY, Bd. 52, Nr. 6, 17. April 1999 (1999-04-17), Seiten a215-a216, XP000989552
- D. M. SCOPE ET AL : "Preliminary report: Use ofsildenafil to treat dyskinesias in patients with Parkinson's disease" NEUROLOGY, Bd. 54, Nr. 7, April 2000 (2000-04), Seiten a90-a91, XP002162785

## Beschreibung

Die vorliegende Erfindung betrifft die Verwendung von Verbindungen der folgenden Formel (I) für die Herstellung eines Arzneimittels zur Behandlung von Neuropathien, wie z.B. peripheren diabetischen Polyneuropathien und Gastroparesen sowie allgemein degenerativen, toxischen, metabolischen, ischämischen und anderen autonomen Formen von Neuropathien im engeren, nämlich neurologischen Sinne.

Es wurde überraschenderweise gefunden, dass sich die z.B. aus WO 93/07149 als solche und zur Verwendung als Arzneimittel für kardiovaskulare Leiden bekannten Verbindungen der Formel (I) 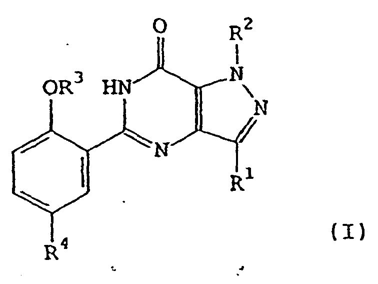 in der
R¹ = C₁₋₆-Alkyl, gegebenenfalls durch Halogen substituiert,
R² = Wasserstoff oder C₁₋₄-Alkyl, gegebenenfalls durch Halogen substituiert
R³ = C₂₋₄-Alkyl, gegebenenfalls durch Halogen substituiert,
R⁴ = SO₂NR⁵R⁶,
   C₁₋₄-Alkyl, gegebenenfalls substituiert mit NR⁵R⁶, CN, CONR⁵R⁶, CO₂R⁷ oder Halogen,
   C₂₋₄-Alkenyl, gegebenenfalls substituiert mit NR⁵R⁶, SONR⁵R⁶, CONR⁵R⁶, CO₂R⁷ oder Halogen,
   C₂₋₄-Alkanoyl, gegebenenfalls substituiert mit NR⁵R⁶, SONR⁵R⁶, CONR⁵R⁶, CO₂R⁷ oder Halogen,
R⁵ und R⁶ unabhängig voneinander Wasserstoff oder C₁₋₄- Alkyl bedeuten oder zusammen mit dem Stickstoffatom, an dem sie hängen, einen Pyrrolidino-, Piperidino-, Morpholino-, 4-(NR⁸)-1-Piperazinyl- oder 1-Imidazolylring bedeuten, der gegebenenfalls mit ein oder zwei C₁₋₄-Alkylgruppen substituiert ist,
R⁷ = Wasserstoff oder C₁₋₄-Alkyl und
R⁸ = Wasserstoff, C₁₋₃-Alkyl oder Hydroxyalkyl mit 1 - 4 C-Atomen bedeutet, sowie die pharmazeutisch akzeptablen Salze solcher Verbindungen (I) zur chemotherapeutischen Behandlung von Neuropathien der oben genannten Art eignen.

Halogen in den obigen Definitionen bedeutet Fluor, Chlor oder Brom, wobei Fluor bevorzugt wird.

Verbindungen entsprechend oder analog dieser Formel einschliesslich ihrer Salze und Verfahren zur Herstellung solcher Verbindungen und Salze sind z.B. aus EP 0 463 756 bekannt, wo sie zur prophylaktischen oder therapeutischen Behandlung von Herz-Kreislauf-Erkrankungen vorgeschlagen wurden. Die kardiovaskulare Aktivität von Verbindungen der Formel (I) beruht darauf, dass diese Verbindungen wirksame und selektive Inhibitoren für die cyclische 3',5'-Monophosphat-phosphodiesterase (cGMP PDE) sind.

Es ist nicht bekannt bzw. auf Grund des Bekannten nicht wahrscheinlich, dass diese Inhibitorwirkung bei Neuropathien der genannten Art eine signifikante Rolle spielt. Die Wirksamkeit von Verbindungen der Formel (I) zur Behandlung von Neuropathien ist tatsächlich auch nicht auf Grund theoretischer Überlegungen sondern auf empirischem Wege festgestellt worden und war weder zu erwarten noch vorauszusehen.

In der nicht vorveröffentlichten EP 1 074 258 ist die Verwendung von Verbindungen der Formel (I) für die Herstellung von Arzneimitteln zur Vorbeugung oder Behandlung von Augenerkrankungen, insbesondere der Optikusneuropathie, beschrieben. Als besonders bevorzugte Substanz wird Sildenafil genannt. Diese Verwendung wird vorliegend ausgeschlossen.

Gegenstand der vorliegenden Erfindung ist daher die Verwendung von Verbindungen der Formel (I) oder/und von deren pharmazeutisch akzeptablen Salzen zur. Herstellung eines Arzneimittels für die therapeutische Behandlung von Neuropathien der oben genannten Art mit Ausnahme von Optikusneuropathien.

Bevorzugte Formen der erfindungsgemässen Verwendung sind in den Patentansprüchen 2 und 3 definiert.

Beispiele für pharmazeutisch akzeptable Salze von Verbindungen und weitere Syntheseverfahren sind ebenfalls aus der oben genannten EP 0 463 756 und ferner aus WO 93/07149 sowie aus WO 93/06104 und WO 94/05661 bekannt.

Zur Herstellung der erfindungsgemässen Arzneimittel können die Wirkstoffe der Formel I in bekannter Art mit den üblichen Adjuvantien und Trägestoffen als feste oder flüssige Mittel konfektioniert werden.

In einer bevorzugten Gruppe von Verbindungen (I) bedeutet R⁴ eine Gruppe der Formel (II) 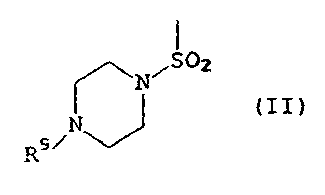 insbesondere dann, wenn R¹, R², R³ und R⁹ jeweils Alkylgruppen mit 1 - 4 C-Atomen, insbesondere Methyl oder Ethyl bedeuten, die gegebenenfalls durch Halogen, insbesondere Fluor, substituiert oder ersetzt sein können.

Solche Verbindungen entsprechen der Formel Ia 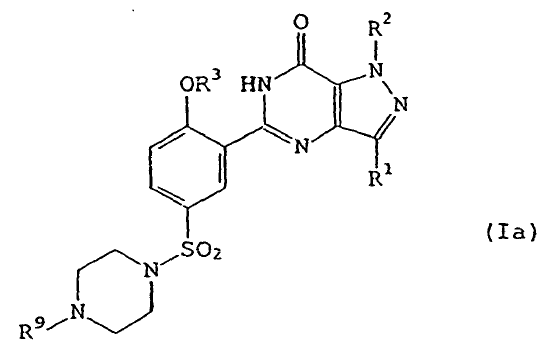 in der die Gruppen R¹ bis R³ und R⁹ die oben angegebene Bedeutung haben.

Eine bevorzugte spezielle Verbindung für die erfindungsgemässe Verwendung entspricht der Formel III 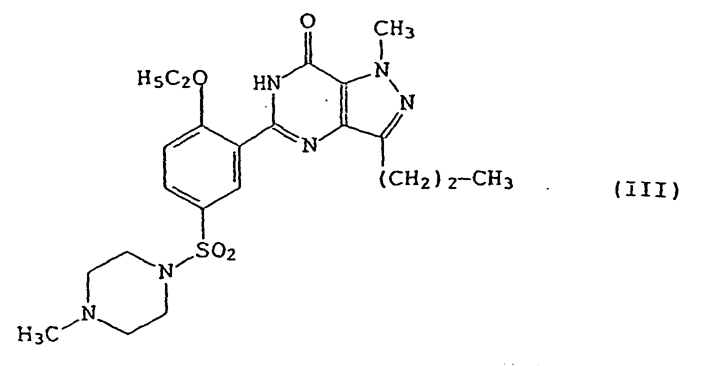 und ist die unter der Freibezeichnung Sildenafil zur Behandlung von Erektionsstörungen bekannte Verbindung.

Verbindungen der Formel (III) und ihre pharmazeutisch akzeptablen Salze können ebenfalls in an sich bekannter Weise, z.B. nach dem in der EP 0 463 756 beschriebenen Verfahren, hergestellt werden.

Es ist zu erwarten, dass die zur Behandlung von Neuropathien wirksamen Dosierungen allgemein in einem ähnlichen oder tieferen Bereich liegen, wie bei bzw, als den bekannten medizinischen Indikationen der Verbindungen (1) bzw. (3), d.h. typisch im Bereich von 1 - 100 mg/Tag, insbesondere 5 - 50 mg/Tag liegen und typisch bei 25 - 50 mg/Woche

Die Erfindung wird anhand von nicht einschränkenden Beispielen weiter erläutert.

### Beispiel 1

Ein männlicher Patient (Alter 66 Jahre) litt seit 9 Jahren an Diabetes mellitus Typ 2. Bei stabil guten Blutzuckerwerten (HbAlc zwischen 6 und 7%) zeigten sich Symptome einer diabetischen Polyneuropathie, nämlich Vibrationssinn 2/8, fehlende Filamentempfindung und abgeschwächte Warm/Kalt-Differenzierung. Wegen einer gleichzeitig bestehenden erektilen Dysfunktion wurde er mit Sildenafil in der kommerziell erhältlichen Zubereitung (Tabletten) mit 50mg/Woche bei einmaliger Verabreichung behandelt.
Zwölf Monate nach Therapiebeginn zeigte sich eine weitgehend normale neurologisache Situation mit Vibrationssinn 5/8, intakter Filamentempfindung und Warm/Kalt-Differenzierung. Subjektiv bemerkte der Patient ein Verschwinden der Temperaturmissempfindung.

### Beispiel 2

Eine 61 jährige Patientin litt seit etwa 35 Jahren an Diabetes mellitus Typ 1. An Komplikationen stellten sich eine Retinopathie und eine schmerzhafte Neuropathie ein. Die Blutzuckerstoffwechsellage befand sich unter intensivierter Insulintherapie im nicht-optimalen Bereich (HbAlc um 8%). Die Patientin Patientin litt dabei an einer schmerzhaften Neuropathie und wurde erfolglos mit verschiedenen üblichen Medikamenten behandelt.
Nach Medikation mit Sildenafil (50 mg/Woche bei jeweils einmaliger Verbreichung der ganzen Wochendosis kam es in den folgenden drei Monate zu einer anhaltenden Verbesserung der Schmerzsymptomatik. Die objektivierbaren Befunde besserten sich ebenfalls.

## Patentansprüche

1. Verwendung einer Verbindung der Formel (I) 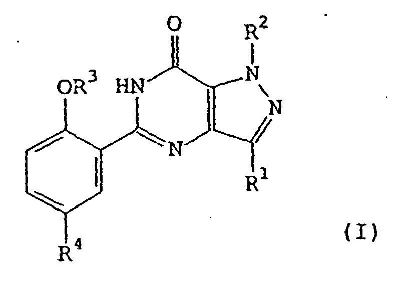 in der
R¹ = C₁₋₆-Alkyl, gegebenenfalls mit Halogen substituiert,
R² = Wasserstoff, C₁₋₄-Alkyl, gegebenenfalls mit Halogen substituiert oder durch Halogen ersetzt,
R³ = C₂₋₄-Alkyl, gegebenenfalls mit Halogen substituiert,
R⁴ = SO₂NR⁵R⁶,
C₁₋₄-Alkyl, gegebenenfalls substituiert mit NR⁵R⁶, CN, CONR⁵R⁶, CO₂R⁷ oder Halogen,
C₂₋₄-Alkenyl, gegebenenfalls substituiert mit NR⁵R⁶, SONR⁵R⁶, CONR⁵R⁶, CO₂R⁷ oder Halogen,
C₂₋₄-Alkanoyl, gegebenenfalls substituiert mit NR⁵R⁶, SONR⁵R⁶, CONR⁵R⁶, CO₂R⁷ oder Halogen,
R⁵ und R⁶ unabhängig voneinander Wasserstoff oder C₁₋₄- Alkyl bedeuten oder zusammen mit dem Stickstoffatom, an dem sie hängen, einen Pyrrolidino-, Piperidino-, Morpholino-, 4-(NR⁸)-1-Piperazinyl- oder 1-Imidazolylring bedeuten, der gegebenenfalls mit ein oder zwei C₁₋₄- Alkylgruppen substituiert ist,
R⁷ = Wasserstoff, C₁₋₄-Alkyl, gegebenenfalls mit Fluor substituiert, und
R⁸ = Wasserstoff, C₁₋₃-Alkyl oder Hydroxyalkyl mit 1 - 4 C-Atomen bedeutet, oder eines pharmazeutisch akzeptablen Salzes einer solchen Verbindung zur Herstellung eines Arzneimittels für die Behandlung von Neuropathien mit Ausnahme von Optikusneuropathien.

2. Verwendung nach Anspruch 1, **dadurch gekennzeichnet, dass** die Verbindung der Formel Formel Ia 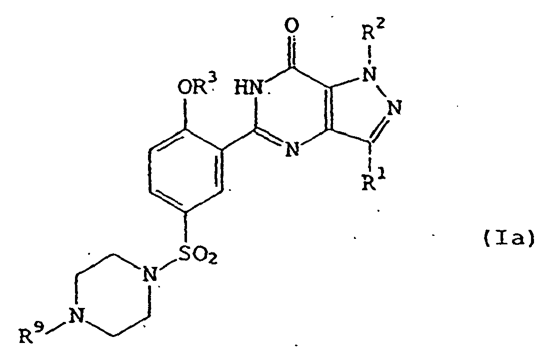 entspricht, in der die Gruppen R¹ bis R³ die in Anspruch 1 angegebene Bedeutung haben und R⁹ eine Alkylgruppe mit 1 - 4 C-Atomen ist, die gegebenenfalls durch Halogen substituiert oder ersetzt ist, oder eines pharmazeutisch akzeptablen Salzes einer solchen Verbindung.

3. Verwendung nach Anspruch I, **dadurch gekennzeichnet, dass** die Verbindung (I) der Formel (III) 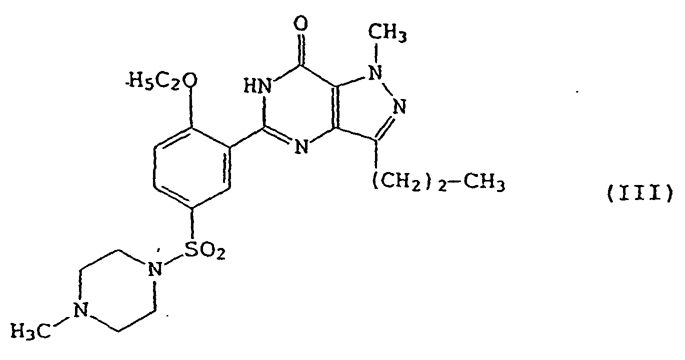 entspricht oder ein pharmazeutisch akzeptables Salz einer solchen Verbindung ist.

## Claims

1. A use of a compound of formula (I): 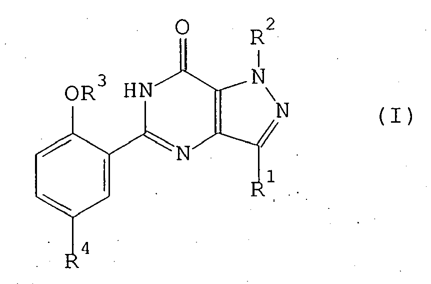 in which:
R¹ = C₁₋₆-alkyl, optionally substituted with halogen,
R² = hydrogen or C₁₋₄-alkyl, optionally substituted with halogen or replaced by halogen,
R³ = C₂₋₄-alkyl, optionally substituted with halogen,
R⁴ = SO₂NR⁵R⁶,
C₁₋₄-alkyl, optionally substituted with NR⁵R⁶, CN, CONR⁵R⁶, CO₂R⁷, or halogen,
C₂₋₄-alkenyl, optionally substituted with NR⁵R⁶, SONR⁵R⁶, CONR⁵R⁶, CO₂R⁷, or halogen,
C₂₋₄alkanoyl, optionally substituted with NR⁵R⁶, SONR⁵R⁶, CONR⁵R⁶, CO₂R⁷, or halogen,
R⁵ and R⁶, independent of one another, represent hydrogen or C₁₋₄-alkyl, or, together with the nitrogen atom to which they are attached, represent a pyrrolidino, piperidino, morpholino, 4-(NR⁸)-1-piperazinyl or 1-imidazolyl ring which, optionally, may be substituted with one or two C₁₋₄-alkyl groups,
R⁷ = hydrogen, C₁₋₄-alkyl, optionally, are substituted with fluorine, and
R⁸ = hydrogen, C₁₋₃-alkyl, or hydroxy alkyl with 1 - 4 C-atoms; or of a pharmaceutically acceptable salt of such a compound for production of a pharmaceutical for treatment of neuropathies;
excluding optic neuropathy.

2. The use according to Claim 1, **characterized in that** the compound (I) complies with formula (Ia): 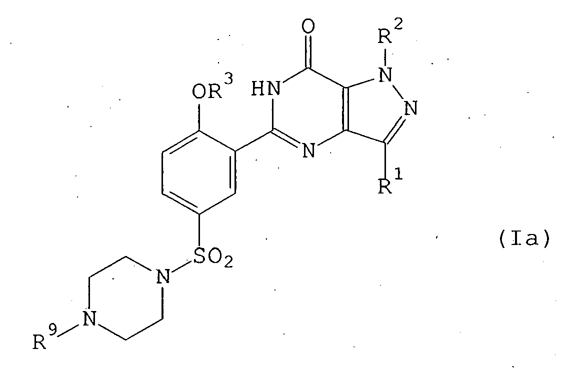 in which the groups R¹ to R³ have the meaning specified in Claim 1, and R⁹ is an alkyl group having 1 - 4 C-atoms which, optionally, are substituted or replaced by halogen; or of a pharmaceutically acceptable salt of such a compound.

3. The use according to Claim 1, **characterized in that** the compound (I) complies with formula (III): 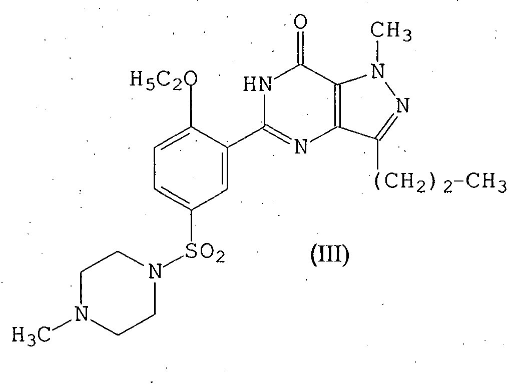 or is a pharmaceutically acceptable salt of such a compound.

## Revendications

1. Utilisation d'un composé de la formule (I) 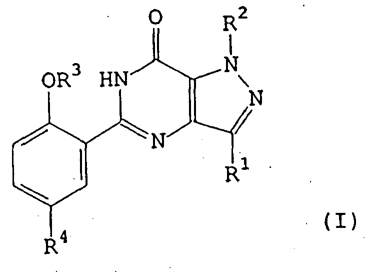 dans laquelle
R¹ = un radical C₁₋₆-alkyle, le cas échéant, substitué par des halogènes,
R² = l'hydrogène, un radical C₁₋₄-alkyle, le cas échéant, substitué par des halogènes ou remplacé par des halogènes,
R³ = un radical C₂₋₄-alkyle, le cas échéant, substitué par des halogènes
R⁴ = SO₂NR⁵R⁶,
C₁₋₄-alkyle, le cas échéant, substitué à l'aide de NR⁵R⁶, CN, CONR⁵R⁶, CO₂R⁷ ou un halogène,
C₂₋₄-alcényle, le cas échéant, substitué à l'aide de NR⁵R⁶, SONR⁵R⁶, CONR⁵R⁶, CO₂R⁷ ou un halogène,
C₂₋₄-alcanoyle, le cas échéant, substitué à l'aide de NR⁵R⁶, SONR⁵R⁶, CONR⁵R⁶, CO₂R⁷ ou un halogène,
R⁵ et R⁶ désignant, indépendamment l'un de l'autre, l'hydrogène ou un radical C₁₋₄₋alkyle ou désignant, conjointement à l'atome d'azote auxquel ils sont attachés, un cycle pyrrolidino-, pipéridino, morpholino-, 4-(NR⁸)-1-pipérazinyl- ou 1-imidazolyl-, qui est, le cas échéant, substitué par un ou deux groupements C₁₋₄-alkyle,
R⁷ = l'hydrogène, un radical C₁₋₄-alkyle, le cas échéant, substitué par un fluor, et
R⁸ = l'hydrogène, un radical C₁₋₃-alkyle ou hydroxyalkyle ayant 1 -4 atomes de C,
ou d'un sel pharmaceutiquement acceptable d'un composé de ce genre, en vue de la fabrication d'un médicament pour le traitement de neuropathies, à l'exception des neuropathies optiques.

2. Utilisation selon la revendication 1, **caractérisée en ce que** le composé correspond à la formule Ia 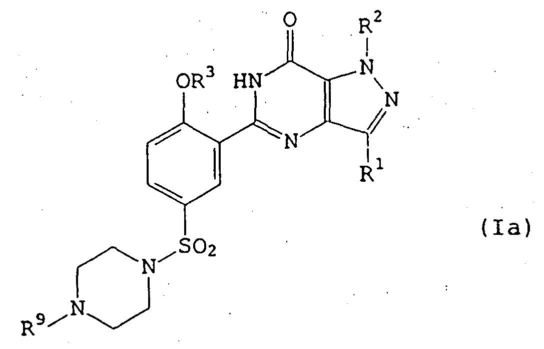 dans laquelle les groupements R¹ à R³ ont la signification indiquée dans la revendication 1 et R⁹ est un groupement alkyle ayant 1 - 4 atomes de C, qui est substitué ou remplacé, le cas échéant, par un halogène ou un sel pharmaceutiquement acceptable d'un composé de ce genre.

3. Utilisation selon la revendication 1, **caractérisée en ce que** le composé (I) correspond à la formule (III) 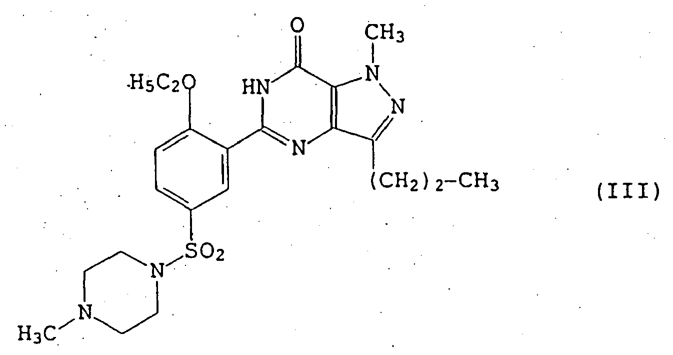 ou est un sel pharmaceutiquement acceptable d'un composé de ce genre.
